# EUROPEAN PATENT SPECIFICATION

(11) **EP 3 340 838 B1**
(45) Date of publication and mention of the grant of the patent: **27.05.2020**
(21) Application number: 16720799.2
(22) Date of filing: 28.04.2016
(51) Int. Cl.: A47F 5/01, A61B 50/20, A61B 42/40

(54) **HOLDING ARRANGEMENT FOR PACKAGES**
HALTEANORDNUNG FÜR VERPACKUNGEN
SYSTÈME DE SUPPORT POUR EMBALLAGES

(30) Priority: 27.08.2015 SE 1551116
(43) Date of publication of application: 04.07.2018
(73) Proprietor: Harry Holms AB, 684 30 Munkfors (SE)
(72) Inventor: HOLM, Jörgen, 684 93 Ransäter (SE); FALKSÄTER, Rickard, 684 92 Munkfors (SE)
(74) Representative: Hynell Intellectual Property AB
(86) International application number: PCT/EP2016/059545
(87) International publication number: WO 2017/032465

(56) References cited:
- WO-A1-2014/148967
- FR-A- 1 569 403
- US-A- 955 589
- US-A- 2 560 896

## Description

### TECHNICAL FIELD

The present invention relates to a holding arrangement for packages according to the first part of claim 1.

### BACKGROUND

There exist a great variety of holding arrangements for packages of different kind. For example, within medical care there is a large use of such holding arrangements for cardboard boxes which contain products, particularly disposable articles such as disposable gloves of for example latex. In medical care clean and hygienic handling is often a requirement.

Different problems relate to the holding of packages for such articles. It is often required that the product in the package be easily accessible, e.g. having the opening conveniently positioned and providing easy removal. Preferably the removal shall be performed one by one of the articles without more articles being withdrawn at the same time, since normally it is not allowed to put anything back into the package due to hygiene requirements. Generally, it is desired that articles in the package should not become dirty or contaminated. This in turn poses some requirements on holding arrangements for such packages.

From WO 0 074 530 there is known a holder arrangement for packages, e.g. for gloves, which is intended for wall mounting. This holder arrangement comprises a number of shelves for boxes and with clamping means that firmly retain a box on each shelve, wherein all the parts are made of wire. Herein the opening of the package will be located in the vertical plane. This known holder arrangement is commonly used and does meet most of the requirements needed. However, the design is rather costly.

From WO2014148967 there is known another holding arrangement for packages, comprising a frame structure connected to a mounting element for vertical mounting of the holding arrangement, one or more holding elements arranged at the frame structure, for reception of and retaining of a package so that access is admitted to articles through an opening therein. Each holding element comprises a support section forming a bottom support plane for the lower side of the package, which forms an angle which exceeds 100 DEG, allowing positioning of a package in each holding element so that its respective side with an opening will be positioned in an obliquely, upwards, outwards inclined plane.

From US3089597 there is known a holding arrangement in accordance with the introductory portion of claim 1. However, this holding arrangement is of a complex kind providing a variety of attachment possibilities which lead to increased cost. Furthermore, the design is not suited for arranging a plurality of holding arrangements within one and the same construction.

A general problem with all of the above known holding arrangements is that the positioning of the boxes over time will lead to an undesired contamination of the articles in a box. Another general problem is that they require two hands to make an exchange of a box in the holder. A further common disadvantage is that it may be difficult to pull one article out of the opening by the use of only one hand, i.e. without use of the other hand to withhold the box.

### SUMMARY OF THE INVENTION

It is therefore an object of the present invention to provide a solution to one or more of the above mentioned problems, and to provide an arrangement through which one or more of the problems can be solved.

A first object is to provide an arrangement which facilitates the maintaining of high demands on cleanness and hygiene at the same time as it is easy to withdraw articles out of a package opening of a package located in the holding arrangement, and that may keep high hygiene requirements over time, which is achieved by a holding arrangement according to claim 1.

Thanks the invention there is provided a holding arrangement that is highly hygienic due to having the opening of the box in a protected position and at the same time that is capable of holding packages in place in such a way that it becomes possible to insert/remove a box without use of both hands, since in the preferred embodiment in a multiple holding arrangement the open section will be freely available for inserting/removing a box, which furthermore may enable a very compact design, since there is no need for extra space to enable inserting/removing a box.

Moreover, according to a specific object of the invention, that may be applied by itself or in combination with other features of the invention, there is arranged an outlet member that may assist in withdrawing articles using but one hand, without the package being tilted or articles unintentionally being withdrawn independently of the filling degree of the package.

Thanks to the design there is furthermore provided an arrangement that is easy and relatively cheap to produce.

It also provides easy adaptation for use with varying needs concerning size, desired amount of packages to be stored in the holding arrangement, available space for positioning of the holding arrangement etc.

Further advantageous may understood from the embodiments indicated by the characteristic features of the dependent claims.

### BREIF DESCRIPTION OF THE DRAWINGS

The invention will in the following be further described, in a non-limiting manner, and with reference to accompanying drawings, in which:
Fig. 1 shows, in perspective, a first embodiment of a holding arrangement according to the invention,
Fig. 2 is a front view of a portion of the holding arrangement in Fig. 1,
Fig. 3 is a side view of a portion of the holding arrangement in Fig. 1.and,
Fig. 4 in perspective view, shows an upper part of alternate embodiment of a holding arrangement according to the invention,

### DETAILED DESCRIPTION

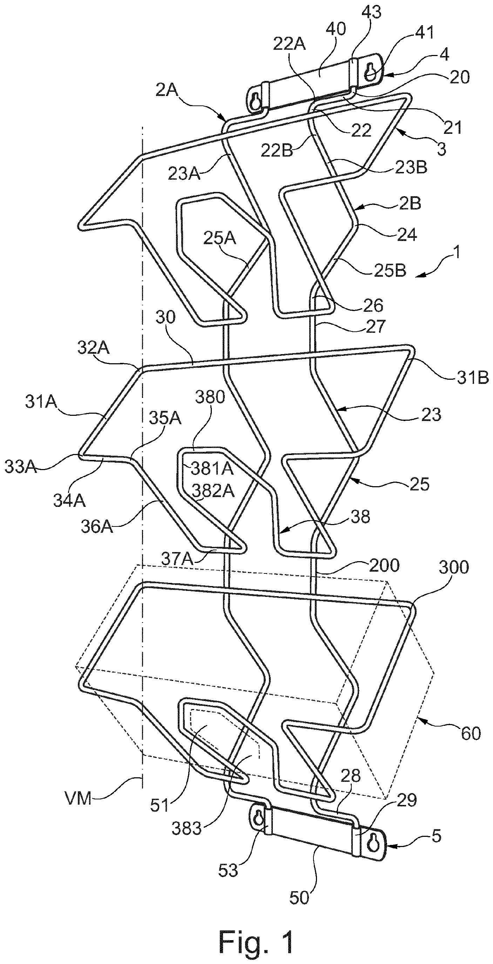

In figures 1, 2 and 3 there is shown a preferred embodiment according to the invention, wherein Fig. 1 in a perspective view presents a holding arrangement 1 according to a preferred kind comprising a plurality of holding members 3, i.e. to enable holding of a plurality of boxes 50 positioned vertically above and underneath each other. It is shown that the support structure 2 is made from two parallel metal rods 200 formed in a repetitive pattern that is the same for support for each one of the holder members 3. Also the holder members 3 are made from metal rods, wherein each metal rod 300 form an endless loop for each one of the holder members 3, having exactly the same configuration for each one of the holder members 3.

Fig. 1 shows a holding arrangement 1 having a frame structure 2 comprising two parallel frame elements 2A, 2B extending in parallel in a substantially vertical direction when the holding arrangement is in a mounted state. In the shown embodiment the frame elements 2A, 2B at their upper ends are mounted (for example detachably introduced into grooves 43 in the mounting element or fixedly connected thereto) at an upper mounting element 4 for example for mounting on a wall by the use of screws (not shown). The mounting element 4 can be formed in any appropriate way for connection to a wall, a rim, a bed etc.

The mounting element may have a dual functionality and also comprise a separate mounting element (not shown) for mounting on a rail or a ramp intended for equipment for intensive care, a so called IVA rail.

In the shown embodiment the frame elements 2A, 2B have free lower ends (here denoted lower since after mounting of the holding arrangement they will be located in its lower part), which are mounted at, mountable at, or in one piece with, a lower mounting element 5. The frame structure 2 here comprises two frame elements 2A, 2Bwith said upper and lower end portions extending in or terminating in, one and the same plane, forming a mounting plane (for example vertical in parallel with a wall plane).

The frame elements here comprise a wire shaped material of stainless steel, but also other materials are of course possible to use.

In ends opposed to the free upper, outer ends of the upper ends portions the frame elements (the wire elements) are angled, at rear support points formed by 90° bends 24A, 24B. Preferably each part 23A, 23B, 25A, 25B to form an acute angle of 45° with the horizontal plane. One part of the frame elements 2A, 2B extend a distance which is a part of and forms a bottom support section 2 which is intended to form a bottom support for the bottom side of a package 50, i.e. the side of the package which is opposite to the side of a package being provided with an opening 51.

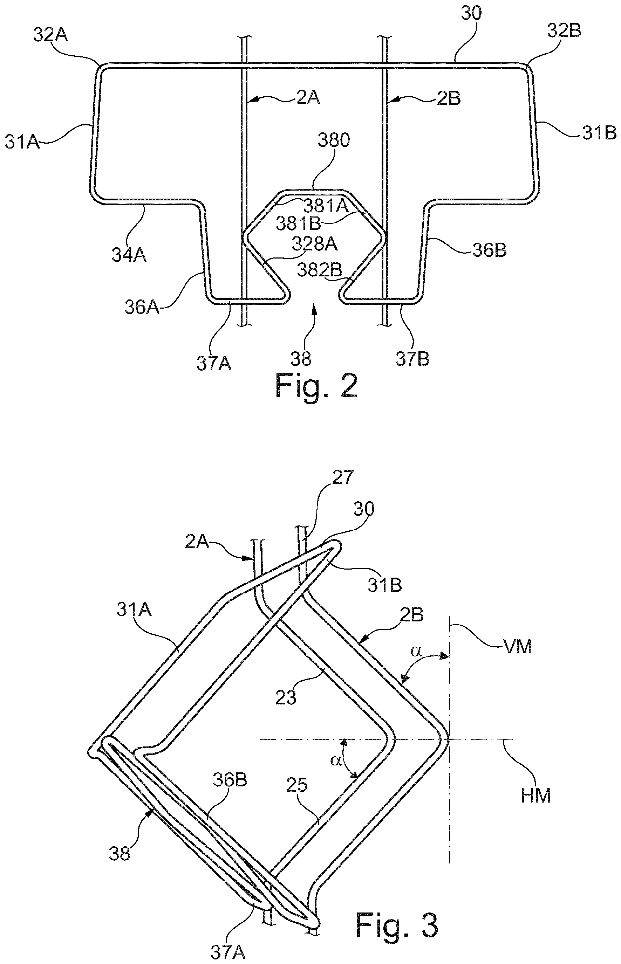

In the shown example the holder arrangement 1 is intended to be vertically attached to a wall by means of attachment members 4, 5, one at the top 4 and one at the bottom 5. In the shown example these are made from plate members 40, 50 having attachment holes 41, 51 and semi cylindrical retaining members 43, 53 to retain end 20, 29 of the support structure 2. Starting from above of the support structure 2 each rod 200 is arranged with a first downwardly and outwardly inclined extending part 21 that via a bend 22A transforms into a substantially vertically extending part 22. In connection with this vertically extending part 22 an upper horizontal part 30 of the holder member 3 is securely (e.g. welding) attached to, and forming a joining bridge between the two parallel positioned support rods 2A, 2B. The vertical support member 22 transforms via a further bend 22B to a downwardly and inwardly inclined member 23 that in turn transforms in to a bend 24, of substantially 90°, thereby again transforming the next part 25 of the rod 200 to extend downwardly and outwardly. The angle α in relation to the horizontal plane (see Fig. 3) is about 45°.

This part 25 of the support structure 2 will form the bottom support of the holder member 3. And the first part 23 will form a part of the back support of the holder member 3. At the end of the bottom support part 25 there is a bend 26 of about 45° that transforms in to a substantially vertically extending part 27, that preferably is in line with the first upper vertical part 22 of the support structure 2. Parts 37A, 37B of the loop 300 form of the front section of the holder member 3 which is attached at an upper location of this vertical part 27. At the lower location of this vertical part 27 the upper horizontal part 30 of the next holder member 3 is attached, in the same manner as described above. Then again there is a bend 27B, having the same function as the bend 22B at the top, transforming the rod in to an inwardly, downwardly directed part 23 of exactly the same kind as described in relation to the first holding member 3.

From here on the support structure 2 will present a repetitive pattern that is repeated for each one of the holder member 3 positioned further down. In connection with the holder member 3 positioned furthest down there is a similar design of the rod 200 as described in a relation to the top, but mirror imaged, i.e. a downwardly directed part 28 extending about 45° in relation to the horizontal plane and a bend 29 of about 45° transforming to a rod end, to fit in to the vertical semi cylinder 53 of the lowest attachment device 5.

As can be seen by Fig. 1 the bends 24 between the bottom support part 25 and the back support part 23, preferably are arranged in the same vertical plane as the attachment members 4, 5, thereby providing support surfaces along the support structure 2 for the holding arrangement 1 against the wall on to which the holding arrangement 1 is attached.

As already mentioned the holder member 3 is completed by the use of the rod 300 presenting an endless loop, wherein there is an upper horizontal part 30, bridging the two parallel rods 200 of the support structure 2. These horizontal upper parts 30, together with the back support part 23 of the support structure 2, forms the back support for a box 60 within a holder member 3. At each end of the upper part 30 there is a bend 32A, 32B of 90°, thereby transforming in to side support parts 31A, 31B parallelly extending at each side, in a downwardly inclined direction, i.e. in parallel with the bottom support part 25 of the support structure 2. The length of a side part 31A, 31B, and also the upper part 30 is adapted to fit the cross sectional size of a box 60 to be retained in the holder member 3. At the lower ends of the side supports 31A, 31B there is again a 90° bend 33A, 33B transforming the rod 300 to extend substantially horizontally, i.e. in parallel with the upper part 30, in an inward direction, thereby forming a first portion of a front section of each holder member 3. At the inner ends of these parts 34A, 34B there is again a 90° bend, where after the rod 300 presents two parallel extending parts 36A, 36B forming further support included in the front section, whereby these parts 36A, 36B extend in parallel with the back support parts 23 of the support structure 2. At the lower end of these parts 36A, 36B, there is again a 90° bend transforming the rod 300 to extend horizontally across each one of the parallel extending support members 2A, 2B, thereby again bridging the two parallel support members 2A, 2A, e.g. by mean of welds.

In accordance with a preferred embodiment of the invention there is arranged an outlet part 38 at the center location of the front section. In accordance with the preferred embodiment as shown in Fig. 1-3 this in a form of an outlet member 38 formed by the same rod 300 forming the other parts included in the holder member 3. It is shown that from the lower parts 37A, 37B of the rod 300 in the holder member 3, at their inner ends there is formed an outlet loop including lower side parts 382A, 382B forming a lower subsection of the outlet 38. Then there are bends of about 120° forming upper side parts 381A, 382B of the outlet 38 and finally an upper part 380 extending substantially horizontally forming the upper end of the outlet 38. The space 383 limited by the outlet 38 is sufficiently large to enclose/cover the outlet opening 61 existing on a box 60 (see Fig. 1) to be positioned within the holder member 3.

Thanks to the use of an outlet member 38 there is arranged a safe positioning of the box also during taking one particle out of the box. Further, thanks to the inclination of the box, i.e. forwardly inclined, the articles within the box 60 will be urged in a direction towards the opening 61 of the box 60 by means of gravity, implying that the user will not need to stick their fingers in to the box to get hold of an article. Moreover, in relation to hygienic requirements, the inclination of the box in a frontward direction will eliminate contamination from small particles moving downwards by means of gravity in the space round the boxes 60, i.e. thanks to having the opening directed downwardly, the back side and the upper side of the box will be located so as to hinder contaminating particles coming from above to move downwardly into the opening 61.

It is an advantage of the invention that secure and hygienic retaining capability of packages 50 in a holding arrangement 1 is provided, as well as that access to articles is facilitated through the angled position, which makes the openings 51 easy to access, despite a hygienic positioning of the opening 51. Furthermore, it provides for articles to lie in close proximity to the opening 51, thanks to the inclination and gravity. It is also an advantage that a concept of holding arrangements is provided which is very flexible and which simply can be produced for different needs and depending on available space, for vertical mounting above each other as well as horizontally next to each other arranged of holding elements in any desired number.

Through its forming the holding arrangement furthermore is compact. It can also be provided with different types of mounting elements which enables mounting on trolleys, benches etc., the mounting elements can be combined for different mounting options, or be dedicated for a particular kind of mounting.

The invention is not limited to the particularly illustrated embodiments, but it can be freely varied within the scope of the appended claims. If the holding elements are detachably arranged, it is also possible to, in one and the same holding arrangement, use holding elements for different dimensions of packages, for example for packages for disposable gloves and packages for masks, aprons or other one way articles which for example are kept in packages of different forms and/or sizes. It is evident that the outlet member 38 may also be arranged in a form of a separate part on to the loop 300 of the holding member 3, e.g. attached to the downwardly extending parts 36A, 36B of the front section of the holder member 3, and also that the holder member parts may be further divided, i.e. formed by a plurality of parts, e.g. rods.

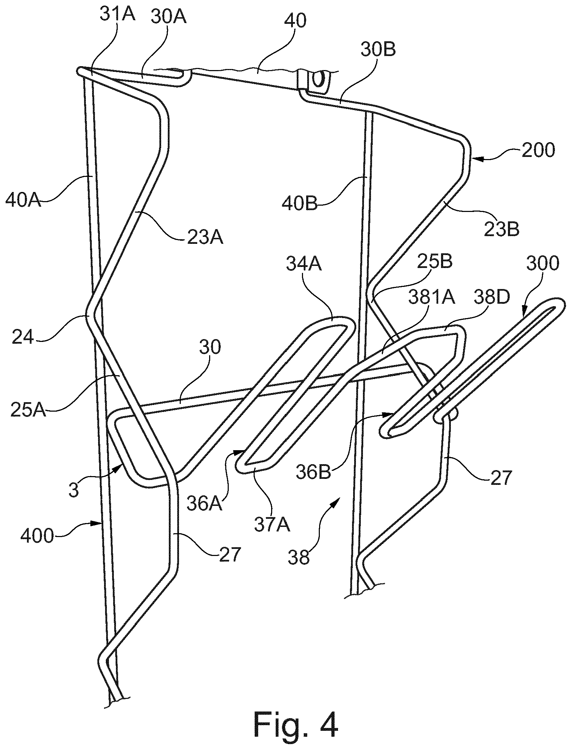

In Fig. 4 there is shown a further embodiment according to the invention. The basic principles are the same as described above and therefore many of the reference numbers are the same as has already been described. Below, there will therefore mainly be focus on differences compared to the above embodiment. A first difference is that for each holding arrangement 3 there are provided merely three support sections 23 A, 23B; 25A, 25B; 36A, 36B. Accordingly, the holding arrangement 3 provides support at the back 23A, 23B, the bottom 25A, 25B and the front 36A, 36B, but provides no support at the side edges. As a consequence, packages of varying length may be used in connection with this holding arrangement 3. Thanks to the use of the outlet arrangement 38 the package will still be centered in connection with taking an article out of the package, as explained above. In order to provide sufficient support, the endless rod 300 that is used for the front section is arranged with an extra downturn 34A', 34B' adjacent the open side sections to facilitate arranging supporting cross-section 30 between the two rod parts 25A, 25B that form the bottom section. This transversal rod part 30 stabilizes the whole construction. Of course it will also be feasible to position the transversal rod in the same plane as the extension of the front section 36A, 36B, i.e. extending transversally between the bends that form the transition between the bottom section 25A, 25B and the intermediate section 27 forming the interconnection with the next holding arrangement 23. However, in most application it is an advantage to position the transversal rod 30 closer to the back side, thereby providing a better support to all parts of the construction.

Another significant difference is the use of vertically extending support rods 40A, 40B, that interconnect the bends 24 of 90° between the back section 23A, 23B and the bottom section 25A, 25B. Thereby a much more stable construction is achieved, which may be especially advantageous if a great many holding arrangements 3 are arranged on top of each other. Of course the stabilizing vertical members 40A, 40B may also be arranged in connection with the embodiment presented in Figs. 1-3.

According to a modification the invention is obtained by using the holder member 3 to form the bottom section. Hence in such a modification only the rear section is formed by the support structure 2A, 2B, i.e. parts 23 as shown in fig 1 and 3 belong to the support structure 2A, 2B and parts 25 are then formed by the rod belonging to the holder member 3. The support structure 2A, 2B may then have a more or less straight extension between an inner, lower end 24 of an upper first rear section and the upper end of an upper second rear section.

Furthermore, it is evident for the skilled person that the functionality in accordance with the invention may also be achieved by use of other devices than rods to produce the frame structure 2A, 2B, e.g. sheet formed plates that are bend in to the desired structure.

Furthermore, it is evident for the skilled person that the outlet member 38 is a device that may also be advantage in connection with other types of holder arrangements, e.g. also prior art known devices such as the one mentioned in the introductory portion of the description, WO2014148967. As a consequence, it is foreseen that one or more divisional applications may be filed, wherein a specific protection will be applied for in regard to the use of an outlet member in connection with holder arrangement of any kind, but preferably in relation to holder arrangement produced mainly by the use of metal rods. In this regard it is evident to the skilled person that such a holder member 38 may take many different forms, especially if separate devices are used to achieve the functionality of said outlet member 38. For instance, it is foreseen that a separate sheet formed device (not shown) may be applied having a hole 380 that provides the limited space providing the desired functionality. In that connection it may be beneficial to have such a sheet to be transparent, thereby allowing the user to be able to look through it, e.g. to take note of any written information on the box and/or to be able to localize the opening of the box. In a specific embodiment such a sheet may be produced in an elastic material, e.g. polyamide, and arranged with snap in openings at the bottom edge thereof to enable attachment to the rods 25 and thereby anchoring in the vertical direction. Moreover, it is foreseen that such a sheet is easily fixated also in the horizontal direction by providing "dents" at the front part of a rod 31A, 31B, in one or both of the side sections, whereby such a sheet will also be fixed within certain limits also regarding horizontal movements.

## Claims

1. Holding arrangement for packages for products, for example disposable articles, comprising a frame structure (2A, 2B) which comprises, or is fixedly or detachably connected to, a mounting element (4, 5) for substantially vertical mounting of the holding arrangement (1) thereby having the frame structure (2A, 2B) extending in a vertical mounting plane (VM), one or more holding members (3) which are fixedly or detachably connected to the frame structure (2A, 2B), wherein each holding member (3) is adapted to delimit a parallelepipedal space to assist in receiving and retaining a parallelepiped package (60) so that access is admitted to products or articles in the package (60) through an opening (61) therein, wherein further each holding member (3) comprises five support sections (23 A, B; 25 A, B, 30; 31 A; 31 B; 36A, 36B) which are arranged to, in one plane each, to form support for said package (60), and leaving one top section open for introduction and removal of the package (60), **characterized in that** said frame structure (2A, 2B), at least partly, forms a back section (23 A, B) and a bottom section (25 A, B), wherein said bottom section (25 A, B) is positioned forming an angle (α) in relation to a horizontal mounting plane (HM) that is perpendicular to said vertical mounting plane (VM), and **in that** a front section (36A, B) is arranged with an outlet member (38) comprising at least an upper part (380) fixedly attached to said front section (36A,B) and arranged to limit an outlet space (383) of said outlet member (38) below said upper part (380).

2. Holding arrangement according to claim 1, **characterized in that** said outlet member (38) forms an integral part with said front section (36A, B).

3. Holding arrangement according to claim 1 or 2, **characterzized** in that said upper part (380) comprises a substantially horizontal portion.

4. Holding arrangement according to any of claims 1-3, **characterized in that** the frame structure (2A, 2B) comprises two or more parallel frame elements (2A, 2B), comprising wire elements of metal, preferably of stainless steel.

5. Holding arrangement according to any one of claims 1-4, **characterized in that** said frame elements (2A, 2B) comprise a 90 degree bend (24 A, B) joining a rear section (23 A, B) and a bottom section (25 A,B) of said holding member (3), wherein preferably said bends (24 A, B) are located in the same plane as the mounting element/s (4, 5) for substantially vertical mounting of the holding arrangement (1).

6. Holding arrangement according to any preceding claim 1, **characterized in** all sections are formed by a wire shaped material of metal, preferably stainless steel or any other appropriate material.

7. Holding arrangement according to claim 6, **characterized in that** one single wire loop (300) is included in forming four of said sections (30; 31 A; 31 B; 36A, 36B).

8. Holding arrangement according to any one of the preceding claims, **characterized in that** said angle (α) is between 30 and 60°, preferably between 40° and 50°, most preferred between 42° and 48°.

9. Holding arrangement according to any one of claims 1-8, **characterized in that** it comprises two or more holding elements (3) .

10. Holding arrangement according to any one of claims 1-9, **characterized in that** the mounting element/s (4,5) comprises a mounting element adapted for mounting on a rail or a ramp for mounting of intensive care equipment.

11. Holding arrangement according to any of claims 1-10, **characterized in that** there are provided five support sections.

12. Method for holding a package of products , for example disposable articles, providing a frame structure (2A, 2B) which comprises, or is fixedly or detachably connected to, a mounting element (4, 5) for substantially vertical mounting of the holding arrangement (1) thereby having the frame structure (2A, 2B) extending in a vertical mounting plane (VM), providing one or more holding members (3) which are fixedly or detachably connected to the frame structure (2A, 2B), wherein each holding member (3) is adapted to delimit a parallelepipedal space to assist in receiving and retaining a parallelepiped package (60) admitting access to products or articles in the package (60) through an opening (61) therein, wherein further each holding element (3) is provided with three support sections (23A, 23B; 25A, 25B, 30; 31A; 31B; 36A, 36B) which are arranged to, in one plane each, to form support for a package, and leaving one top section open for introduction and removal of the package (60), **characterized by** providing said frame structure (2A, 2B), at least partly, to form a back section (23A, 23B) and a bottom section (25A, 25B), wherein said bottom section (25A, 25B) is positioned to form an angle (α) in relation to a horizontal mounting plane (HM) that is perpendicular to said vertical mounting plane (VM), and by providing a front section (36A, B) with an outlet member (38) comprising at least an upper part (380) fixedly attached to said front section (36A,B) and arranged to limit an outlet space (383) of said outlet member (38) below said upper part (380), enabling to position the package (60) to have the side provided with the opening (61) in a downwards facing plane in a mounted state of the holding arrangement.

13. Method according to claim 12, **characterized by** proving said outlet member (38) to form an integral part with said front section (36A, 36B).

14. Method according to claim 12 or 13, **characterized by** providing said upper part (380) in the form of a substantially horizontal portion.

## Patentansprüche

1. Halteanordnung für Verpackungen für Produkte, beispielsweise Einwegartikel, umfassend eine Rahmenstruktur (2A, 2B), die eine Befestigungskomponente (4, 5) zur im Wesentlichen vertikalen Befestigung der Halteanordnung (1) umfasst, oder fest oder abnehmbar damit verbunden ist, wodurch sich die Rahmenstruktur (2A, 2B) in einer vertikalen Befestigungsebene (VM) erstreckt, ein oder mehrere Halteelemente (3), die fest oder abnehmbar mit der Rahmenstruktur (2A, 2B) verbunden sind, wobei jedes Halteelement (3) dazu angepasst ist, einen quaderförmigen Raum abzugrenzen, um das Aufnehmen und Halten einer quaderförmigen Verpackung (60) zu unterstützen, so dass der Zugang zu Produkten oder Artikeln in der Verpackung (60) durch eine Öffnung (61) darin gewährt wird, wobei ferner jedes Halteelement (3) fünf Stützbereiche (23A, B; 25A, B, 30; 31A; 31B; 36A, 36B) umfasst, die so angeordnet sind, dass sie jeweils in einer Ebene eine Stütze für die Verpackung (60) bilden und einen oberen Bereich zum Einführen und Entfernen der Verpackung (60) offen lassen, **dadurch gekennzeichnet, dass** die Rahmenstruktur (2A, 2B) zumindest teilweise einen hinteren Bereich (23A, B) und einen unteren Bereich (25A, B) bildet, wobei der untere Bereich (25A, B) so positioniert ist, dass er einen Winkel (α) in Bezug auf eine horizontale Befestigungsebene (HM) bildet, die senkrecht zu der vertikalen Befestigungsebene (VM) ist, und dass ein vorderer Bereich (36A, B) mit einem Auslasselement (38) angeordnet ist, welches zumindest einen oberen Teil (380) umfasst, der fest an dem vorderen Bereich (36A, B) angebracht und so angeordnet ist, dass er einen Auslassraum (383) des Auslasselements (38) unterhalb des oberen Teils (380) begrenzt.

2. Halteanordnung nach Anspruch 1, **dadurch gekennzeichnet, dass** das Auslasselement (38) einen integralen Bestandteil des vorderen Bereichs (36A, B) bildet.

3. Halteanordnung nach Anspruch 1 oder 2, **dadurch gekennzeichnet, dass** der obere Teil (380) einen im Wesentlichen horizontalen Abschnitt umfasst.

4. Halteanordnung nach einem der Ansprüche 1 bis 3, **dadurch gekennzeichnet, dass** die Rahmenstruktur (2A, 2B) zwei oder mehr parallele Rahmenkomponenten (2A, 2B) umfasst, die Drahtkomponenten aus Metall, vorzugsweise aus Edelstahl, umfassen.

5. Halteanordnung nach einem der Ansprüche 1 bis 4, **dadurch gekennzeichnet, dass** die Rahmenkomponenten (2A, 2B) eine 90-Grad-Biegung (24A, B) umfassen, welche einen hinteren Bereich (23A, B) und einen unteren Bereich (25A, B) des Halteelements (3) verbindet, wobei vorzugsweise sich die Biegungen (24A, B) in derselben Ebene wie die Befestigungskomponente bzw. die Befestigungskomponenten (4, 5) befinden, um die Halteanordnung (1) im Wesentlichen vertikal zu befestigen.

6. Halteanordnung nach einem der vorangehenden Ansprüche, **dadurch gekennzeichnet, dass** alle Bereiche aus einem drahtförmigen Material aus Metall, vorzugsweise aus Edelstahl, oder einem anderen geeigneten Material gebildet sind.

7. Halteanordnung nach Anspruch 6, **dadurch gekennzeichnet, dass** eine einzelne Drahtschleife (300) in die Bildung von vier der Bereiche (30; 31A; 31B; 36A, 36B) einbezogen ist.

8. Halteanordnung nach einem der vorangehenden Ansprüche, **dadurch gekennzeichnet, dass** der Winkel (α) zwischen 30 und 60° liegt, vorzugsweise zwischen 40° und 50°, am meisten bevorzugt zwischen 42° und 48°.

9. Halteanordnung nach einem der Ansprüche 1 bis 8, **dadurch gekennzeichnet, dass** sie zwei oder mehr Halteelemente (3) umfasst.

10. Halteanordnung nach einem der Ansprüche 1 bis 9, **dadurch gekennzeichnet, dass** die Befestigungskomponente bzw. die Befestigungskomponenten (4,5) eine Befestigungskomponente umfasst bzw. umfassen, die zur Befestigung auf einer Schiene oder einer Rampe zur Befestigung von Intensivpflegegeräten angepasst ist.

11. Halteanordnung nach einem der Ansprüche 1 bis 10, **dadurch gekennzeichnet, dass** fünf Stützbereiche vorgesehen sind.

12. Verfahren zum Halten einer Verpackung von Produkten, beispielsweise Einwegartikeln, umfassend das Bereitstellen einer Rahmenstruktur (2A, 2B), die eine Befestigungskomponente (4, 5) zur im Wesentlichen vertikalen Befestigung der Halteanordnung (1) umfasst, oder fest oder abnehmbar damit verbunden ist, wodurch sich die Rahmenstruktur (2A, 2B) in einer vertikalen Befestigungsebene (VM) erstreckt, das Bereitstellen von einem oder mehreren Halteelementen (3), die fest oder abnehmbar mit der Rahmenstruktur (2A, 2B) verbunden sind, wobei jedes Halteelement (3) dazu angepasst ist, einen quaderförmigen Raum abzugrenzen, um das Aufnehmen und Halten einer quaderförmigen Verpackung (60) zu unterstützen, so dass der Zugang zu Produkten oder Artikeln in der Verpackung (60) durch eine Öffnung (61) darin gewährt wird, wobei ferner jedes Halteelement (3) mit drei Stützbereichen (23A, 23B; 25A, 25B, 30; 31A; 31B; 36A, 36B) versehen ist, die so angeordnet sind, dass sie jeweils in einer Ebene eine Stütze für die Verpackung bilden und einen oberen Bereich zum Einführen und Entfernen der Verpackung (60) offen lassen, **gekennzeichnet durch** das Bereitstellen der Rahmenstruktur (2A, 2B) zumindest teilweise in einer Weise, dass sie einen hinteren Bereich (23A, 23B) und einen unteren Bereich (25A, 25B) bildet, wobei der untere Bereich (25A, 25B) so positioniert ist, dass er einen Winkel (α) in Bezug auf eine horizontale Befestigungsebene (HM) bildet, die senkrecht zu der vertikalen Befestigungsebene (VM) ist, und durch das Bereitstellen eines vorderen Bereichs (36A, B) mit einem Auslasselement (38), welches zumindest einen oberen Teil (380) umfasst, der fest an dem vorderen Bereich (36A, B) angebracht und so angeordnet ist, dass er einen Auslassraum (383) des Auslasselements (38) unterhalb des oberen Teils (380) begrenzt, wodurch es ermöglicht wird, in einem befestigten Zustand der Halteanordnung die Verpackung (60) so zu positionieren, dass sich die mit der Öffnung (61) versehene Seite in einer nach unten weisenden Ebene befindet.

13. Verfahren nach Anspruch 12, **gekennzeichnet durch** das Bereitstellen des Auslasselements (38) in einer Weise, dass es einen integralen Teil des vorderen Bereichs (36A, 36B) bildet.

14. Verfahren nach Anspruch 12 oder 13, **gekennzeichnet durch** das Bereitstellen des oberen Teils (380) in Form eines im Wesentlichen horizontalen Abschnitts.

## Revendications

1. Agencement de maintien pour des emballages de produits, par exemple des articles à usage unique, comprenant une structure (2A, 2B) formant ossature qui comprend un élément de montage (4, 5) ou qui est reliée de manière fixe ou amovible à celui-ci, pour un montage sensiblement vertical de l'agencement de maintien (1), la structure (2A, 2B) formant ossature s'étendant ainsi dans un plan de montage vertical (VM), un ou plusieurs éléments de maintien (3) qui sont reliés de manière fixe ou détachable à la structure (2A, 2B) formant ossature, chaque élément de maintien (3) étant adapté pour délimiter un espace parallélépipédique pour aider à recevoir et à retenir un emballage parallélépipédique (60) de sorte que soit permis un accès à des produits ou des articles présents dans l'emballage (60) à travers une ouverture (61) aménagée dans celui-ci, chaque élément de maintien (3) comprenant en outre cinq sections de support (23 A, B ; 25 A, B, 30 ; 31 A ; 31 B ; 36A, 36B) qui sont agencées pour, chacune dans un plan, former un support pour ledit emballage (60), et laissant une section supérieure ouverte pour l'introduction et le retrait de l'emballage (60), **caractérisé en ce que** ladite structure (2A, 2B) formant ossature, au moins partiellement, forme une section arrière (23 A, B) et une section inférieure (25 A, B), ladite section inférieure (25 A, B) étant positionnée en formant un angle (a) par rapport à un plan de montage horizontal (HM) qui est perpendiculaire audit plan de montage vertical (VM), et **en ce qu'**une section avant (36A, B) est agencée avec un élément de sortie (38) comprenant au moins une partie supérieure (380) reliée de manière fixe à ladite section avant (36A, B) et agencée pour limiter un espace de sortie (383) dudit élément de sortie (38) en dessous de ladite partie supérieure (380).

2. Agencement de maintien selon la revendication 1, **caractérisé en ce que** ledit élément de sortie (38) forme une partie intégrante avec ladite section avant (36A, B).

3. Agencement de maintien selon la revendication 1 ou la revendication 2, **caractérisé en ce que** ladite partie supérieure (380) comprend une partie sensiblement horizontale.

4. Agencement de maintien selon l'une quelconque des revendications 1 à 3, **caractérisé en ce que** la structure (2A, 2B) formant ossature comprend deux ou plusieurs éléments d'ossature parallèles (2A, 2B), comprenant des éléments en fil métallique, de préférence en acier inoxydable.

5. Agencement de maintien selon l'une quelconque des revendications 1 à 4, **caractérisé en ce que** lesdits éléments d'ossature (2A, 2B) comprennent un coude à 90 degrés (24 A, B) reliant une section arrière (23 A, B) et une section inférieure (25 A, B) dudit élément de maintien (3), lesdits coudes (24 A, B) étant de préférence situés dans le même plan que le ou les éléments de montage (4, 5) pour un montage sensiblement vertical de l'agencement de maintien (1).

6. Agencement de maintien selon l'une quelconque des revendications précédentes, **caractérisé en ce que** toutes les sections sont formées par un matériau en forme de fil métallique, de préférence en acier inoxydable ou en tout autre matériau approprié.

7. Agencement de maintien selon la revendication 6, **caractérisé en ce qu'**une seule boucle de fil (300) est incluse dans la formation de quatre desdites sections (30 ; 31 A; 31 B ; 36A, 36B).

8. Agencement de maintien selon l'une quelconque des revendications précédentes, **caractérisé en ce que** ledit angle (a) est compris entre 30 et 60°, de préférence entre 40° et 50°, et de façon encore préférée entre 42° et 48°.

9. Agencement de maintien selon l'une quelconque des revendications 1 à 8, **caractérisé en ce qu'**il comprend deux ou plusieurs éléments de retenue (3).

10. Agencement de maintien selon l'une quelconque des revendications 1 à 9, **caractérisé en ce que** le ou les éléments de montage (4, 5) comprennent un élément de montage adapté pour un montage sur un rail ou une rampe en vue du montage d'un équipement de soins intensifs.

11. Agencement de maintien selon l'une quelconque des revendications 1 à 10, **caractérisé en ce qu'**il est prévu cinq sections de support.

12. Procédé pour maintenir un emballage de produits, par exemple des articles jetables, incluant le fait de prévoir une structure (2A, 2B) formant ossature qui comprend un élément de montage (4, 5), ou est reliée de manière fixe ou détachable à celui-ci, pour un montage sensiblement vertical de l'agencement de maintien (1), la structure (2A, 2B) formant ossature s'étendant ainsi dans un plan de montage vertical (VM), le fait de prévoir un ou plusieurs éléments de maintien (3) qui sont reliés de manière fixe ou détachable à la structure (2A, 2B) formant ossature, chaque élément de maintien (3) étant adapté pour délimiter un espace parallélépipédique afin d'aider à recevoir et à retenir un emballage parallélépipédique (60) permettant l'accès à des produits ou des articles présents dans l'emballage (60) à travers une ouverture (61) dans celui-ci, chaque élément de maintien (3) étant en outre muni de trois sections de support (23A, 23B ; 25A, 25B, 30 ; 31A ; 31B ; 36A, 36B) qui sont agencées pour, chacune dans un plan, former un support pour un emballage, et laisser une section supérieure ouverte pour l'introduction et le retrait de l'emballage (60), **caractérisé par** le fait de prévoir ladite structure (2A, 2B) formant ossature, au moins partiellement, de façon à former une section arrière (23A, 23B) et une section inférieure (25A, 25B), ladite section inférieure (25A, 25B) étant positionnée pour former un angle (a) par rapport à un plan de montage horizontal (HM) qui est perpendiculaire audit plan de montage vertical (VM), et par le fait de prévoir une section avant (36A, B) avec un élément de sortie (38) comprenant au moins une partie supérieure (380) reliée de manière fixe à ladite section avant (36A, B) et agencée pour limiter un espace de sortie (383) dudit élément de sortie (38) en dessous de ladite partie supérieure (380), permettant de positionner l'emballage (60) de façon que le côté pourvu de l'ouverture (61) se trouve dans un plan orienté vers le bas dans un état monté de l'agencement de maintien.

13. Procédé selon la revendication 12, **caractérisé par** le fait de prévoir ledit élément de sortie (38) de façon qu'il forme une seule pièce avec ladite section avant (36A, 36B).

14. Procédé selon la revendication 12 ou la revendication 13, **caractérisé par** le fait de prévoir ladite partie supérieure (380) sous la forme d'une portion sensiblement horizontale.
